# EUROPEAN PATENT APPLICATION

(11) **EP 2 527 841 A1**
(43) Date of publication of application: **28.11.2012**
(21) Application number: 11167420.6
(22) Date of filing: 25.05.2011
(51) Int. Cl.: G01N 33/564, C07K 14/47

(54) **Methods for the diagnosis of rheumatoid arthritis**

(71) Applicant: Toscana Biomarkers S.r.l., 53100 Siena (IT)
(72) Inventor: Pratesi, Federico, 53100 Siena (IT); Chelli, Mario, 53100 Siena (IT); Lolli, Francesco, 53100 Siena (IT); Paolini, Ilaria, 53100 Siena (IT); Papini, Anna Maria, 53100 Siena (IT); Alcaro, Maria Claudia, 53100 Siena (IT); Migliorini, Paola, 53100 Siena (IT); Rovero, Paolo, 53100 Siena (IT)
(74) Representative: Capasso, Olga

(57) **Abstract**

The present invention refers to the use of citrullinated synthetic peptides for the diagnosis of rheumatoid arthritis (RA).

## Description

### Field of invention

The present invention refers to the use of citrullinated synthetic peptides for the diagnosis of rheumatoid arthritis (RA). In particular, the invention relates to the use of peptide sequences that specifically react with autoantibodies present in the sera of RA patients.

### State of the art

In the broad spectrum of inflammatory joint diseases, rheumatoid arthritis (RA) has a prominent position. By causing symmetrical and destructive inflammation in the small and large joints, RA leads to pain and joint failure, eventually resulting in disfiguration and disability.

The occurrence of various autoantibodies is a hallmark of RA. The best known RA biomarker is the rheumatoid factor (RF), a class of IgG, IgA or IgM antibodies directed against the Fc-region of the IgG isotype of immunoglobulins. About 70-80% of RA-patients are sero-positive for RF, but RFs are also found in chronic infections, lymphoproliferative diseases, other rheumatic diseases, and in nearly 20% of elderly healthy individuals. Thus, given the limited specificity of RF for RA, other more specific antibodies have been sought. APF (anti-perinuclear factor) antibodies and AKAs (anti-keratin antibodies) were clearly demonstrated to be highly disease specific.

The antigens recognized by APF antibodies and AKAs were later found to be citrulline-containing proteins post-translationally generated by modifying arginines into citrulline residues. The discovery of the involvement of citrullinated epitopes in RA boosted the identification of such highly specific RA biomarkers. Anti-citrullinated proteins/peptides antibodies (ACPA) are a family of biomarkers present almost exclusively in RA [G.J. Pruijn et al. Arthritis Res. Ther. 2010, 12, 203]. Because of their high disease-specificity, ACPA have been recently included in the serological criteria for the classification and diagnosis of RA [D. Aletaha et al. Ann. Rheum. Dis. 2010, 69, 1580]. Different citrullinated peptides and proteins have been used to set up specific and sensitive RA diagnostic assays recognizing ACPA [US20020143143, US20070087380, EP1946109, US20070148704, WO2009007846, WO2009000077, WO2007017556, EP1456662, WO1999028344, WO2008132264, WO2010115745, WO2010117694, US2011065609]. Among all the proteins associated with RA, the following citrullinated sequences have been used in diagnostic assays: fibrin [C. Masson-Bessière et al. J. lmmunol. 2001, 166, 4177], collagen II [H. Burkhardt et al. Eur. J. lmmunol. 2005, 35, 1643], vimentin [E.R. Vossenaar et al. Arthritis Rheum. 2004, 50, 3485], filaggrin [US20090028885, US7445903, US6890720], and viral proteins [C. Anzilotti et al. J. Rheumatol. 2006, 33, 647; WO2004087747]. The first ELISA using synthetic citrullinated peptides (CPs) derived from filaggrin [G.A. Schellekens et al. J. Clin. Invest. 1998, 101, 273]. To increase the sensitivity of the test, the CPs were modified to a structure, the cyclic citrullinated peptide (CCP), in which the citrulline moiety is optimally exposed for antibody binding [WO1998022503]. In a so-called CCP1 test [G.A. Schellekens et al. Arthritis Rheum. 2000, 43, 155], antibodies could be detected in 68% of sera from RA patients with a specificity of 98% with a single CCP. Recent studies indicate that the second generation of CCP test, CCP2 [J. Avouac et al. Ann. Rheum. Dis. 2006, 65, 845], is very sensitive and specific, showing a sensitivity of 68% (range 39-94%) with a specificity of 95% (range 81-100%). Recently, a third-generation assay was developed by INOVA [L. Lutteri et al. Clin. Chim. Acta 2007, 386, 76] and claimed to be a new more sensitive anti-CCP ELISA which maintains a specificity of 98%. These ELISA tests are all commercially available (Euro-Diagnostica, Arnhem, The Netherlands; Axis-Shield, Dundee, Scotland; INOVA, San Diego, USA).

Recently, the use of citrullinated sequences derived from proteins EBNA1 (VCP1) and EBNA2 (VCP2) of Epstein-Barr virus allowed the set up of diagnostic assays characterized by specificities and sensitivities comparable to the ones reported by CCP2 and CCP3 tests [F. Pratesi et al. Arthritis Rheum. 2006, 54, 733; C. Anzilotti et al. J. Rheumatol. 2006, 33, 647; F. Pratesi et al. Clin. Exp. Immunol. 2011, 164, 337; W02004087747; EP2325195]. Interestingly, the comparison of the performances of the tests based on CCP2, CCP3, VCP1, and VCP2 suggested that different citrullinated sequences can detect different subgroups of ACPA in the same set of patients. These results confirmed the important role of the amino acid sequence surrounding citrulline residues in the formation of anti-CP epitopes, in contrast with the hypothesized exclusive role of the XG or XnonG unit (X = citrulline) [EP1693673].

### EBNA1-peptides

Citrullinated peptides based on the deiminated EBNA1 (35-58) are the object of the international application W02004087747 in which their use to detect antibodies in RA sera is described. This application also discloses that the use of multiple antigen peptide [MAP; J.P. Tam PNAS U.S.A. 1988, 85, 5409], containing at least 4 copies of the deiminated EBNA1(35-58) as coating antigen in immunological assays for the detection of ACPA. Antibodies specific for a peptide corresponding to the EBNA1(35-58) sequence (viral citrullinated peptide 1, VCP1), containing citrulline in place of arginine, were detected in 50% of RA sera and in less than 5% of normal and disease control sera [F. Pratesi et al. Arthritis Rheum. 2006, 54, 733]. The frequency of anti-VCP1 antibodies was determined in 627 serum samples, 300 from patients with RA and 327 from controls, including connective tissue diseases, chronic arthritides, and healthy donors. Anti-VCP1 antibodies were found in 45% of RA sera versus less than 5% of controls [C. Anzilotti et al. J. Rheumatol. 2006, 33, 647].

### EBNA2-peptides

RA involves both genetic and environmental influences and viruses have long been suspected to promote the development of RA. To be likely candidates for a causal role in RA, viruses must be ubiquitous, persist within the body, show direct or indirect tropism for the joints, and be capable of altering host immune responses. The Epstein Barr virus (EBV) exhibits these characteristics. As a result, potential links between EBV and RA have been a focus of research for the last three decades [K.H. Costenbader et al. Arthritis Res. Ther. 2006, 8, 204]. It is known that patients with RA possess elevated levels of antibodies to latent and replicative EBV proteins and in particular to EBNA1. Moreover, the peripheral blood EBV load in RA patients is higher than in controls. A number of studies shed light on the possible etiological role of EBV in RA synovitis [S. Sawada et al. Autoimmun. Rev. 2005, 4, 106]. These studies led to the idea of exploring the immune response in RA patients using CP of viral origin. The best results were obtained with citrullinated peptides derived from the deiminated peptide EBNA2(338-358) (G Q S R G Q S R G R G R G R G R G R G K G, SEQ ID No. 4).

Anti-VCP2 (MAP of formula XII) IgG antibodies were found in 67/104 (64%), IgM in 48/104 (46%) and IgA in 41/104 (40%) of the serum samples of patients with RA, and in less than 5% of normal healthy subjects and disease controls [Pratesi et al. Clin. Exp. Immunol. 2011, 164, 337; EP2325195].

Another class of proteins associated with RA diagnosis is represented by histone proteins.

### H4-peptides

Histones are constituents of the nucleosomes and are classified into five classes: H1, H2A, H2B, H3, and H4. Sequences derived from H1 and H2B were used to detect autoantibodies in systemic lupus erythematosus, RA and systemic sclerosis [W003044054]. Even if it is known that these proteins are natively deiminated and generally modified [K. Arita et al. PNAS 2006, 103, 5291; A.J.W. Zendman et al. Anal. Biochem. 2007, 369, 232], no histone citrullinated sequences have been, up to now, associated with RA. However, microarrays based on the use of modified histones (i.e. phosphorylated, methylated, acetylated, and ubiquitinated), but not citrullinated, were described for the detection of antibodies [W007132177].

An epitope mapping of the full protein H4 showed that the differently citrullinated N-terminal part, H4(1-50), corresponds to the antigenic portion of the molecule [EP10168270.6]. In particular, the citrullinated C-terminal portion of H4(1-50) is highly reactive and can be used to recognize ACPA in RA sera. Anti-H4 IgG antibodies were found in 28% of RA patients using H4(1-20), 65% with H4(14-34) and 60% with H4(30-50).

Noteworthy, the antibodies anti-citrullinated H4 sequences are highly specific to RA, thus belonging to the ACPA family, and are not present in other autoimmune diseases (e.g. systemic lupus erythematosus, systemic sclerosis), in which on the contrary anti-histone antibodies have been detected [W003044054; R. Hesselstrand et al., Rheumatol 2003, 42, 534].

However, there is the need for diagnostic methods and kits of rheumatoid arthritis that are highly sensitive and specific for this autoimmune disease.

### Summary of the invention

The peptides of the invention are defined as follows:

### H4-peptides

The peptides consisting of a sequence being at least 15 amino acid long and comprised in the sequence of: 
-S G X1 G K G G K G L G K G G A K X2 H X3 K V L X4 D N I Q G I T K P A I X5 X6 L
A X7 X8 G G V K X9 I S G L I (SEQ ID No. 1, Formula I);
 In particular, the peptides consist of:
-S G X₁ G K G G K G L G K G G A K X₂ H X₃ (aa 1 to aa 20 of SEQ ID No. 1)
-G A K X₂ H X₃ K V L X₄ D N I Q G I T K P A I (aa 14 to aa 34 of SEQ ID No.1)
-K P A I X₅ X₆ L A X₇ X₈ G G V K X₉ I S G L I (aa 31 to aa 50 of SEQ ID No. 1)
wherein the amino acids X₁-X₉ are selected independently from an arginine residue (Arg) or a citrulline (Cit) residue and at least two of X₁-X₉ are a citrulline residue;
Preferred H4-peptides comprising two citrulline residues are:
-G A K Cit H R K V L Cit D N I Q G I T K P A I (aa 14 to aa 34 of SEQ ID No.1
wherein X₂ = X₄ = Cit and X₃ = Arg)
-K P A I Cit X₆ L A X₇ Cit G G V K X₉ I S G L I (aa 31 to aa 50 of SEQ ID No. 1
wherein X₅ = X₈ = Cit and X₆, X₇ and X₉ = are selected independently from an arginine residue (Arg) or a citrulline (Cit) residue)
-K P A I Cit R L A R Cit G G V K R I S G L I (aa 31 to aa 50 of SEQ ID No. 1
wherein X₅ = X₈ = Cit and X₆ = X₇ = X₉ = Arg)
-K P A I R Cit L A R Cit G G V K R I S G L I (aa 31 to aa 50 of SEQ ID No. 1
wherein X₆ = X₈ = Cit and X₅ = X₇ = X₉ = Arg);
Preferred H4-peptides in which all of X₁-X₉ are citrulline residues are: 
-S G Cit G K G G K G L G K G G A K Cit H Cit K V L Cit D N I Q G I T K P A I Cit
Cit L A Cit Cit G G V K Cit I S G L I (SEQ ID No. 1 wherein X1 = X2= X3= X4 = X5
=X6 = X7=X8 =X9= Cit, Formula II),
 -S G Cit G K G G K G L G K G G A K Cit H Cit K (aa 1 to aa 20 of SEQ ID No. 1 wherein X₁ = X₂ = X₃ = Cit, Formula III),
-S G Cit G K G G K G L G K G G A (aa 1 to aa 15 of SEQ ID No. 1 wherein X₁ = Cit),
-G A K Cit H Cit K V L Cit D N I Q G I T K P A I (aa 14 to aa 34 of SEQ ID No.1 wherein X₂ = X₃ = X₄ = Cit, Formula IV),
-G A K Cit H Cit K V L Cit D N I Q G (aa 14 to aa 28 of SEQ ID No.1 wherein X₂ = X₃ = X₄ = Cit),
-Cit K V L Cit D N I Q G I T K P A I Cit Cit L A (aa 19 to aa 38 of SEQ ID No. 1 wherein X₃ = X₄ = X₅ = X₆ = Cit),
-L Cit D N I Q G I T K P A I Cit Cit L A Cit Cit G (aa 22 to aa 41 of SEQ ID No. 1 wherein X₄ = X₅ = X₆ = X₇ = X₈ = Cit),
-K P A I Cit Cit L A Cit Cit G G V K Cit I S G L I (aa 31 to aa 50 of SEQ ID No. 1 wherein X₅ = X₆ = X₇ = X₈ = X₉ = Cit, Formula V);
-I Cit Cit L A Cit Cit G G V K Cit I S G (aa 34 to aa 48 of SEQ ID No. 1 wherein X₅ = X₆ = X₇ = X₈ = X₉ = Cit);
Preferred H4-peptides in MAP format are: 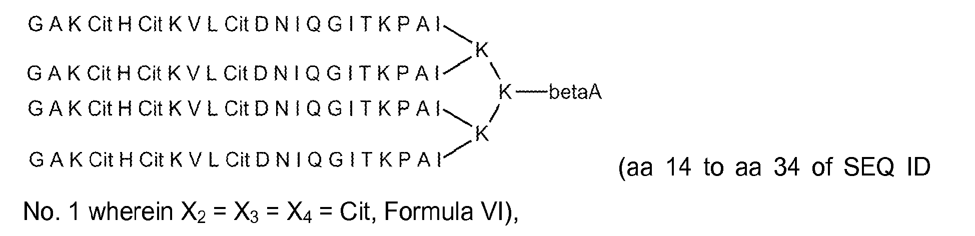
GAKCitHCitKVLCitDNIQGITKPAI
GAKCitHCitKVLCitDNIQGITKPAI
GAKCitHCitKVLCitDNIQGITKPAI
GAKCitHCitKVLCitDNIQGITKPAI
K
K
K
betaA
(aa 14 to aa 34 of SEQ ID
No. 1 wherein X2 = X3 = X4 = Cit, Formula VI),
 
GAKCitHCitKVLCitDNIQGITKPAI
GAKCitHCitKVLCitDNIQGITKPAI
GAKCitHCitKVLCitDNIQGITKPAI
GAKCitHCitKVLCitDNIQGITKPAI
K
K—betaA—C
K
(aa 14 to aa 34 of SEQ
ID No. 1 wherein X2 = X3 = X4 = Cit, Formula VII),
 
KPAICitCitLACitCitGGVKCitISGLI
K
KPAICitCitLACitCitGGVKCitISGLI
K —— betaA
KPAICitCitLACitCitGGVKCitISGLI
K
KPAICitCitLACitCitGGVKCitISGLI
(aa 31 to aa 50 of SEQ ID
No. 1 wherein X5 = X6 = X7 = X8 = X9 = Cit, Formula VIII)
 
KPAICitCitLACitCitGGVKCitISGLI
K
KPAICitCitLACitCitGGVKCitISGLI
K—betaA—C
KPAICitCitLACitCitGGVKCitISGLI
K
KPAICitCitLACitCitGGVKCitISGLI
(aa 31 to aa 50 of SEQ
ID No. 1 wherein X5 = X6 = X7 = X8 = X9 = Cit, Formula IX)
 wherein Cit is a citrulline residue, betaA is a beta-alanine residue and C is a cysteine residue.

### EBNA2-peptides

The peptides consist of a sequence being at least 8 amino acid long and comprised in the sequence of:
G Q S X₁ G Q S X₂ G X₃ G X₄ G X₅ G X₆ G X₇ G K G (SEQ ID No. 2, Formula X),
wherein the amino acids X₁-X₇ are selected independently from an arginine residue or a citrulline residue, and at least one of X₁-X₇ is a citrulline residue;
Preferable peptides consist of:
-G Q S X₁ G Q S X₂ (aa 1 to aa 8 of SEQ ID No. 2);
-G Q S X₁ G Q S X₂ G (aa 1 to aa 9 of SEQ ID No. 2);
-G X₃ G X₄ G X₅ G X₆ G X₇ G K G (aa 9 to aa 21 of SEQ ID No. 2)
wherein the amino acids X₁-X₇ are selected independently from an arginine residue or a citrulline residue, and at least one of X₁-X₇ is a citrulline residue;
Preferred EBNA2-peptides comprising one citrulline residue are:
-G Q S R G Q S Cit G R G R G R G R G R G K G (SEQ ID No. 2 wherein X₂ = Cit and X₁ = X₃ = X₄ = X₅ = X₆ = X₇ = Arg)
-G Q S R G Q S R G R G Cit G R G R G R G K G (SEQ ID No. 2 wherein X₄ = Cit and X₁ = X₂ = X₃ = X₅ = X₆ = X₇ = Arg);
Preferred EBNA2-peptides comprising at least two citrulline residues are:
-G Q S R G Q S Cit G R G Cit G R G R G R G K G (SEQ ID No. 2 wherein X₂ = X₄ = Cit and X₁ = X₃ = X₅ = X₆ = X₇ = Arg)
-G Q S Cit G Q S Cit G R G Cit G R G R G R G K G (SEQ ID No. 2 wherein X₁ = X₂ = X₄ = Cit and X₃ = X₅ = X₆ = X₇ = Arg)
-G Q S Cit G Q S Cit G Cit G Cit G R G R G R G K G (SEQ ID No. 2 wherein X₁ = X₂ = X₃ = X₄ = Cit and X₅ = X₆ = X₇ = Arg)
-G Q S Cit G Q S Cit G R G Cit G Cit G R G R G K G (SEQ ID No. 2 wherein X₁ = X₂ = X₄ = X₅ = Cit and X₃ = X₆ = X₇ = Arg)
-G Q S Cit G Q S Cit G Cit G Cit G Cit G R G R G K G (SEQ ID No. 2 wherein X₁ = X₂ = X₃ = X₄ = X₅ = Cit and X₆ = X₇ = Arg)
-G Q S Cit G Q S Cit G Cit G Cit G R G Cit G R G K G (SEQ ID No. 2 wherein X₁ = X₂ = X₃ = X₄ = X₆ = Cit and X₅ = X₇ = Arg)
-G Q S Cit G Q S Cit G Cit G Cit G Cit G Cit G R G K G (SEQ ID No. 2 wherein X₁ = X₂ = X₃ = X₄ = X₅ = X₆ = Cit and X₇ = Arg)
-G Q S Cit G Q S Cit G Cit G Cit G R G Cit G Cit G K G (SEQ ID No. 2 wherein X₁ =X₂=X₃=X₄=X₆=X₇= Cit and X₅ = Arg);
Preferred EBNA2-peptides in which all of X₁-X₇ are citrulline residues are:
-G Q S Cit G Q S Cit G Cit G Cit G Cit G Cit G Cit G K G (SEQ ID No. 2 wherein all of X₁-X₇ are citrulline residues, Formula XI);
-G Q S Cit G Q S Cit (aa 1 to aa 8 of SEQ ID No. 2 wherein all of X₁-X₂ are citrulline residues);
-G Q S Cit G Q S Cit G (aa 1 to aa 9 of SEQ ID No. 2 wherein all of X₁-X₂ are citrulline residues);
-GCitGCitGCitGCitGCitGKG(aa9toaa21 of SEQ ID No. 2 wherein all of X₃-X₇ are citrulline residues);
Preferred EBNA2-peptides in MAP format are: 
G Q S Cit G Q S Cit G Cit G Cit G Cit G Cit G Cit G K G
G Q S Cit G Q S Cit G Cit G Cit G Cit G Cit G Cit G K G
K
K —— betaA
G Q S Cit G Q S Cit G Cit G Cit G Cit G Cit G Cit G K G
G Q S Cit G Q S Cit G Cit G Cit G Cit G Cit G Cit G K G
K
(SEQ ID No. 2 wherein all
of X1-X7 are citrulline residues, Formula XII)
 
G Q S Cit G Q S Cit G Cit G Cit G Cit G Cit G Cit G K G
K
G Q S Cit G Q S Cit G Cit G Cit G Cit G Cit G Cit G K G
K —betaA—C
G Q S Cit G Q S Cit G Cit G Cit G Cit G Cit G Cit G K G
K
G Q S Cit G Q S Cit G Cit G Cit G Cit G Cit G Cit G K G
(SEQ ID No. 2 wherein all of
X1-X7 are citrulline residues, Formula XIII)
 wherein Cit is a citrulline residue, betaA is a beta-alanine residue and C is a cysteine residue.

### EBNA1-peptides

The peptides consist of a sequence being at least 12 amino acid long and comprised in the sequence of:
G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue;
Preferred EBNA1-peptides in which all of X₁-X₆ are citrulline residues are:
-G G D N H G Cit G Cit G Cit G Cit G Cit G G G Cit P G A P G (SEQ ID No. 3 wherein all of X₁-X₆ are citrulline residues, Formula XV);
-G Cit G Cit G Cit G Cit G Cit G G (aa 6 to aa 17 of SEQ ID No. 3 wherein all of X₁-X₅ are citrulline residues);
-G Cit G Cit G Cit G Cit G Cit G G G Cit P G (aa 6 to aa 21 of SEQ ID No. 3 wherein all of X₁-X₆ are citrulline residues);
Preferred EBNA1-peptides in MAP format are: 
GGDNHGCitGCitGCitGCitGCitGGGCitPGAPG
GGDNHGCitGCitGCitGCitGCitGGGCitPGAPG
K
K
betaA
GGDNHGCitGCitGCitGCitGCitGGGCitPGAPG
GGDNHGCitGCitGCitGCitGCitGGGCitPGAPG
K
(SEQ ID No. 3 wherein all
of X1-X6 are citrulline residues, Formula XVI)
 
GGDNHGCitGCitGCitGCitGCitGGGCitPGAPG
K
GGDNHGCitGCitGCitGCitGCitGGGCitPGAPG
K—betaA—C
GGDNHGCitGCitGCitGCitGCitGGGCitPGAPG
K
GGDNHGCitGCitGCitGCitGCitGGGCitPGAPG
(SEQ ID No. 3
wherein all of X1-X6 are citrulline residues, Formula XVII)
 wherein Cit is a citrulline residue, betaA is a beta-alanine residue and C is a cysteine residue.

In the present invention it was surprisingly found that methods using sequences of human histone H4 peptides in combination with sequences of EBNA2 and/or EBNA1 proteins of Epstein Barr virus (EBV) are very specific and sensitive for the diagnosis of RA by the detection of ACPA in sera of patients.

### Peptides combinations

In the method for the diagnosis of RA of the present invention, at least two peptides are used wherein
-at least one peptide is selected from the group consisting of:
- H4-peptides
and at least one peptide is selected from the group consisting of:
- EBNA2-peptides and/or
- EBNA1-peptides;
the peptides being defined as above.

The method of the invention is performed using a preferred combination comprising:
- one H4-peptide and one EBNA2-peptide **[Combination A];**
- or one H4-peptide and one EBNA1-peptide **[Combination B];**
- or one H4-peptide and two EBNA2-peptides **[Combination C];**
- or two H4-peptides and one EBNA2-peptide **[Combination D];**
- or two H4-peptides and one EBNA1-peptide **[Combination E];**
- or two H4-peptide and two EBNA2-peptides **[Combination F];**
- or one H4-peptide, one EBNA2-peptide, and one EBNA1-peptide **[Combination G];**
- or two H4-peptides, one EBNA2-peptide, and one EBNA1-peptide **[Combination H].**

Still preferably the method is performed using a preferred combination comprising:
- peptide of formula IV or one of its MAP derivative (Formula VI or VII) and peptide of formula XI or one of its MAP derivative (Formula XII or XIII) **[Combination A1].**
- or peptide of formula V or one of its MAP derivative (Formula VIII or IX) and peptide of formula XI or one of its MAP derivative (Formula XII or XIII)

### [Combination A2].

- or peptide of formula IV or one of its MAP derivative (Formula VI or VII) and peptide of formula XV or one of its MAP derivative (Formula XVI or XVII)

### [Combination B1].

- or peptide of formula V or one of its MAP derivative (Formula VIII or IX) and peptide of formula XV or one of its MAP derivative (Formula XVI or XVII)

### [Combination B2].

- or peptide of formula V or one of its MAP derivative (Formula VIII or IX) and peptide of formula IV or one of its MAP derivative (Formula VI or VII) in combination with peptide of formula XI or one of its MAP derivative (Formula XII or XIII)

### [Combination D1].

- or peptide of formula V or one of its MAP derivative (Formula VIII or IX) and peptide of formula IV or one of its MAP derivative (Formula VI or VII) in combination with peptide of formula XV or one of its MAP derivative (Formula XVI or XVII)

### [Combination E1].

- or peptide of formula IV or one of its MAP derivative (Formula VI or VII) and peptide of formula XI or one of its MAP derivative (Formula XII or XIII) in combination with peptide of formula XV or one of its MAP derivative (Formula XVI or XVII) **[Combination G1].**
- or peptide of formula V or one of its MAP derivative (Formula VIII or IX) and peptide of formula XI or one of its MAP derivative (Formula XII or XIII) in combination with peptide of formula XV or one of its MAP derivative (Formula XVI or XVII) **[Combination G2].**
- or peptide of formula V or one of its MAP derivative (Formula VIII or IX) and peptide of formula XI or one of its MAP derivative (Formula XII or XIII) in combination with peptide of formula XV or one of its MAP derivative (Formula XVI or XVII) and with peptide of formula IV or one of its MAP derivative (Formula VI or VII) **[Combination H1].**

Still preferably the method is performed using a preferred combination comprising:
- formula VI (or VII) and formula XII (or XIII) **[Combination A1a].**
- formula VIII (or IX) and formula XII (or XIII) **[Combination A2a].**
- formula VI (or VII) and formula XVI (or XVII) **[Combination B1a].**
- formula VIII (or IX) and formula XVI (or XVII) **[Combination B2a].**
- formula VIII (or IX) and formula VI (or VII) and formula XII (or XIII) **[Combination D1a].**
- formula VIII (or IX) and formula VI (or VII) and formula XVI (or XVII) **[Combination E1a].**
- formula VI (or VII) and formula XII (or XIII) in combination with formula XVI (or XVII) **[Combination G1a].**
- formula VIII (or IX) and formula XII (or XIII) in combination with MAP formula XVI (or XVII) **[Combination G2a].**
- formula VIII (or IX) and formula XII (or XIII) in combination with formula XVI (or XVII) and formula VI (or VII) **[Combination H1a].**

Preferably, the peptide as indicated above is of linear form or of multimeric form. Higher sensitivities and specificities of the method are obtained when multimeric forms of the peptides are used for the antibody recognition. Multimeric forms can be obtained through the conjugation of multiple copies of the peptides to a chemical core (e.g. multiple antigen peptides, MAPs; scaffolded peptides; peptide-carrier conjugates) or by polymerization. In certain embodiments the multimeric form may be a tetravalent MAP. The tetravalent MAP preferably consists of:
- a MAP nucleus structure;
- a linear peptide having an amino acid sequence of formula I-V, X-XI, and/or XIV-XV linked through a chemical bond to each of amino terminal residues of the MAP nucleus structure, wherein the linear peptides are equal or different each others.

According to the invention a MAP nucleus structure is: 
(Yn4)
(Xm) (Yn3)
(Yn4)
(Xm)
(Yn4)
(Xm) (Yn3) (Yn2)
(Yn4)
X (Yn1) Cz
(Yn4)
(Xm) (Yn3) (Yn2)
(Yn4)
(Xm)
(Yn4)
(Xm) (Yn3)
(Yn4)
 wherein X is an amino acid having at least two amino functional residues, Y is an amino acid selected from the group of alanine, beta-alanine, and/or glycine and/or a molecule used as spacer, C is a cysteine, m is 0 or 1, n₁ n₂ n₃ n₄ are integer numbers comprised between 0 and 10, z is 0 or 1, and wherein bonds are carbamido bonds.

A MAP or conjugate comprising a plurality of copies of a citrullinated linear synthetic peptide or of an antigenically effective fragment derived therefrom as described above, bound to an immunologically inert amino-acid core, therefore also falls within the scope of the present invention.

It is therefore an object of the invention an in vitro method for the diagnosis of rheumatoid arthritis in a subject comprising the step of detecting antibodies specific for rheumatoid arthritis in a biological sample by:
a) reacting under proper conditions said biological sample with at least two peptides to produce a peptide-antibody complex, wherein
   - at least one peptide consists of a sequence being at least 15 amino acid long and comprised in the sequence: 
S G X1 G K G G K G L G K G G A K X2 H X3 K V L X4 D N I Q G I T K P A I X5 X6 L
A X7 X8 G G V K X9 I S G L I (SEQ ID No. 1, Formula I)
 wherein the amino acids X₁-X₉ are selected independently from an arginine residue or a citrulline residue and at least two of X₁-X₉ are a citrulline residue
      and
   - at least one peptide is selected from the group of:
      i) a peptide consisting of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
         and/or
      ii) a peptide consisting of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue;
b) detecting the peptide-antibody complex.

Preferably the biological sample is reacted with at least two peptides, wherein
-at least one peptide consists of the sequence:
S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue.

In a preferred embodiment the biological sample is reacted with at least two peptides, wherein
-at least one peptide consists of the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue.

In a still preferred embodiment the biological sample is reacted with at least two peptides, wherein at least one peptide consists of the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue.

In a yet preferred embodiment the biological sample is reacted with:
a) two peptides, wherein
   -one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -one peptide is selected from the group of:
      i) a peptide consisting of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
         or
      ii) a peptide consisting of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue or
b) three peptides, wherein
   -one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -two peptides consist of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
      or
c) three peptides, wherein
   -two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -one peptide is selected from the group of:
      i) a peptide consisting of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
         or
      ii) a peptide consisting of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue
         or
d) three peptides, wherein
   -one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -one peptide consists of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
      and
   -one peptide consists of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue
      or
e) four peptides, wherein
   -two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -two peptides consist of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
      or
f) four peptides, wherein
   -two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
      and
   -one peptide consists of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue and
   -one peptide consists of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue.

In a preferred embodiment the at least one peptide consisting of a sequence being at least 15 amino acid long and comprised in the sequence of SEQ ID No. 1 is selected from the group of:
-S G X₁ G K G G K G L G K G G A (aa 1 to aa 15 of SEQ ID No. 1),
-S G X₁ G K G G K G L G K G G A K X₂ H X₃ (aa 1 to aa 20 of SEQ ID No. 1)
-G A K X₂ H X₃ K V L X₄ D N I Q G (aa 14 to aa 28 of SEQ ID No. 1),
-G A K X₂ H X₃ K V L X₄ D N I Q G I T K P A I (aa 14 to aa 34 of SEQ ID No. 1)
-X₃ K V L X₄ D N I Q G I T K P A I X₅ X₆ L A (aa 19 to aa 38 of SEQ ID No. 1),
-L X₄ D N I Q G I T K P A I X₅ X₆ L A X₇ X₈ G (aa 22 to aa 41 of SEQ ID No. 1),
-K P A I X₅ X₆ L A X₇ X₈ G G V K X₉ I S G L I (aa 31 to as 50 of SEQ ID No. 1)
or
-I X₅ X₆ L A X₇ X₈ G G V K X₉ I S G (aa 34 to aa 48 of SEQ ID No. 1)
wherein the amino acids X₁-X₉ are selected independently from an arginine residue or a citrulline residue and at least two of X₁-X₉ are a citrulline residue.
Preferably the peptide is selected from the group of:
-G A K Cit H R K V L Cit D N I Q G I T K P A I (aa 14 to aa 34 of SEQ ID No. 1 wherein X₂ = X₄ = Cit and X₃ = Arg)
-K P A I Cit X₆ L A X₇ Cit G G V K X₉ I S G L I (aa 31 to aa 50 of SEQ ID No. 1 wherein X₅ = X₈ = Cit and X₆, X₇ and X₉ = are selected independently from an arginine residue (Arg) or a citrulline (Cit) residue)
-K P A I Cit R L A R Cit G G V K R I S G L I (aa 31 to aa 50 of SEQ ID No. 1 wherein X₅ = X₈ = Cit and X₆ = X₇ = X₉ = Arg)
-K P A I R Cit L A R Cit G G V K R I S G L I (aa 31 to aa 50 of SEQ ID No. 1 wherein X₆ = X₈ = Cit and X₅ = X₇ = X₉ = Arg)
-S G Cit G K G G K G L G K G G A K Cit H Cit K V L Cit D N I Q G I T K P A I Cit Cit L A Cit Cit G G V K Cit I S G L I (SEQ ID No. 1 wherein X₁ = X₂= X₃= X₄ = X₅ =X₆ = X₇=X₈ =X₉= Cit, Formula II)
-S G Cit G K G G K G L G K G G A K Cit H Cit K (aa 1 to aa 20 of SEQ ID No. 1 wherein X₁ = X₂ = X₃ = Cit, Formula III),
-G A K Cit H Cit K V L Cit D N I Q G I T K P A I (aa 14 to aa 34 of SEQ ID No. 1 wherein X₂ = X₃ = X₄ = Cit, Formula IV),
-K P A I Cit Cit L A Cit Cit G G V K Cit I S G L I (aa 31 to aa 50 of SEQ ID No. 1 wherein X₅ = X₆ = X₇ = X₈ = X₉ = Cit, Formula V)
-S G Cit G K G G K G L G K G G A (aa 1 to aa 15 of SEQ ID No. 1 wherein X₁ = Cit),
-G A K Cit H Cit K V L Cit D N I Q G (aa 14 to aa 28 of SEQ ID No. 1 wherein X₂ = X₃ = X₄ = Cit), -Cit K V L Cit D N I Q G I T K P A I Cit Cit L A (aa 19 to aa 38 of SEQ ID No. 1 wherein X₃ = X₄ = X₅ = X₆ = Cit),
-L Cit D N I Q G I T K P A I Cit Cit L A Cit Cit G (aa 22 to aa 41 of SEQ ID No. 1 wherein X₄ = X₅ = X₆ = X₇ = X₈ = Cit) or
-I Cit Cit L A Cit Cit G G V K Cit I S G (aa 34 to aa 48 of SEQ ID No. 1 wherein X₅ = X₆ = X₇ = X₈ = X₉ = Cit).
In a preferred embodiment the at least one peptide consisting of a sequence being at least 8 amino acid long and comprised in the sequence of SEQ ID No. 2 is selected from the group of:
-G Q S R G Q S Cit G R G R G R G R G R G K G (SEQ ID No. 2 wherein X₂ = Cit and X₁ = X₃ = X₄ = X₅ = X₆ = X₇ = Arg)
-G Q S R G Q S R G R G Cit G R G R G R G K G (SEQ ID No. 2 wherein X₄ = Cit and X₁ = X₂ = X₃ = X₅ = X₆ = X₇ = Arg)
-G Q S R G Q S Cit G R G Cit G R G R G R G K G (SEQ ID No. 2 wherein X₂ = X₄ = Cit and X₁ = X₃ = X₅ = X₆ = X₇ = Arg)
-G Q S Cit G Q S Cit G R G Cit G R G R G R G K G (SEQ ID No. 2 wherein X₁ = X₂ = X₄ = Cit and X₃ = X₅ = X₆ = X₇ = Arg)
-G Q S Cit G Q S Cit G Cit G Cit G R G R G R G K G (SEQ ID No. 2 wherein X₁ = X₂ = X₃ = X₄ = Cit and X₅ = X₆ = X₇ = Arg)
-G Q S Cit G Q S Cit G R G Cit G Cit G R G R G K G (SEQ ID No. 2 wherein X₁ = X₂ = X₄ = X₅ = Cit and X₃ = X₆ = X₇ = Arg)
-G Q S Cit G Q S Cit G Cit G Cit G Cit G R G R G K G (SEQ ID No. 2 wherein X₁ = X₂ = X₃ = X₄ = X₅ = Cit and X₆ = X₇ = Arg)
-G Q S Cit G Q S Cit G Cit G Cit G R G Cit G R G K G (SEQ ID No. 2 wherein X₁ = X₂ = X₃ = X₄ = X₆ = Cit and X₅ = X₇ = Arg)
-G Q S Cit G Q S Cit G Cit G Cit G Cit G Cit G R G K G (SEQ ID No. 2 wherein X₁ = X₂ = X₃ = X₄ = X₅ = X₆ = Cit and X₇ = Arg)
-G Q S Cit G Q S Cit G Cit G Cit G R G Cit G Cit G K G (SEQ ID No. 2 wherein X₁ = X₂ = X₃ = X₄ = X₆ = X₇ = Cit and X₅ = Arg) or
-G Q S Cit G Q S Cit G Cit G Cit G Cit G Cit G Cit G K G (SEQ ID No. 2 wherein all of X₁-X₇ are citrulline residues Formula XI)
-G Q S Cit G Q S Cit (aa 1 to aa 8 of SEQ ID No. 2 wherein all of X₁-X₂ are citrulline residues);
-G Q S Cit G Q S Cit G (aa 1 to aa 9 of SEQ ID No. 2 wherein all of X₁-X₂ are citrulline residues);
-G Cit G Cit G Cit G Cit G Cit G K G (aa 9 to aa 21 of SEQ ID No. 2 wherein all of X₃-X₇ are citrulline residues).

In a preferred embodiment the at least one peptide consisting of a sequence being at least 12 amino acid long and comprised in the sequence of SEQ ID No. 3 is selected from the group of:
-G X₁ G X₂ G X₃ G X₄ G X₅ G G (aa 6 to aa 17 of SEQ ID Neo. 3) wherein the amino acids X₁-X₉ are selected independently from an arginine residue or a citrulline residue and at least one of X₁-X₅ is a citrulline residue;
-G X₁ G X₂ G X₃ G X₄ G X₅ G G G X₆ P G (aa 6 to aa 21 of SEQ ID No. 3) wherein the amino acids X₁-X₆ are selected independently from an arginine residue or a citrulline residue and at least one of X₁-X₅ is a citrulline residue;
-G Cit G Cit G Cit G Cit G Cit G G (aa 6 to aa 17 of SEQ ID No. 3 wherein all of X₁-X₅ are citrulline residues);
-G Cit G Cit G Cit G Cit G Cit G G G Cit P G (aa 6 to aa 21 of SEQ ID No. 3 wherein all of X₁-X₆ are citrulline residues);
-G G D N H G Cit G Cit G Cit G Cit G Cit G G G Cit P G A P G (SEQ ID No. 3 wherein all of X₁-X₆ are citrulline residues, Formula XV).

Preferably the biological sample is reacted with a combination of:
- G A K Cit H Cit K V L Cit D N I Q G I T K P A I (aa 14 to aa 34 of SEQ ID No. 1 wherein X₂ = X₃ = X₄ = Cit
   and/or
- K P A I Cit Cit L A Cit Cit G G V K Cit I S G L I (aa 31 to aa 50 of SEQ ID No. 1 wherein X₅ = X₆ = X₇ = X₈ = X₉ = Cit
   and
- G Q S Cit G Q S Cit G Cit G Cit G Cit G Cit G Cit G K G (SEQ ID No. 2 wherein all of X₁-X₉ are citrulline residues
   and/or
- G G D N H G Cit G Cit G Cit G Cit G Cit G G G Cit P G A P G (SEQ ID No. 3 wherein all of X₁-X₆ are citrulline residues

Still preferably the peptides of the invention are in a map form: (peptide)₄ K₂ K betaA or (peptide)₄ K₂ K betaA-C wherein betaA is a beta-alanine residue and C is a cysteine residue.
Preferably at least one peptide is selected from the group of: 
GAKCitHCitKVLCitDNIQGITKPAI
K
GAKCitHCitKVLCitDNIQGITKPAI
K —betaA
GAKCitHCitKVLCitDNIQGITKPAI
K
GAKCitHCitKVLCitDNIQGITKPAI
 or 
GA K Cit H Cit K V L Cit D N I Q G I T K P A I
K
GA K Cit H Cit K V L Cit D N I Q G I T K P A I
K —betaA—C
GA K Cit H Cit K V L Cit D N I Q G I T K P A I
K
GA K Cit H Cit K V L Cit D N I Q G I T K P A I
 or 
KPAICitCitLACitCitGGVKCitISGLI
K
KPAICitCitLACitCitGGVKCitISGLI
K —betaA
KPAICitCitLACitCitGGVKCitISGLI
K
KPAICitCitLACitCitGGVKCitISGLI
 or 
KPAICitCitLACitCitGGVKCitISGLI
K
KPAICitCitLACitCitGGVKCitISGLI
K—betaA—C
KPAICitCitLACitCitGGVKCitISGLI
K
KPAICitCitLACitCitGGVKCitISGLI
 or 
G Q S Cit G Q S Cit G Cit G Cit G Cit G Cit G Cit G K G
G Q S Cit G Q S Cit G Cit G Cit G Cit G Cit G Cit G K G
K
K
betaA
G Q S Cit G Q S Cit G Cit G Cit G Cit G Cit G Cit G K G
K
G Q S Cit G Q S Cit G Cit G Cit G Cit G Cit G Cit G K G
 or 
G Q S Cit G Q S Cit G Cit G Cit G Cit G Cit G Cit G K G
K
G Q S Cit G Q S Cit G Cit G Cit G Cit G Cit G Cit G K G
K —betaA—C
G Q S Cit G Q S Cit G Cit G Cit G Cit G Cit G Cit G K G
K
G Q S Cit G Q S Cit G Cit G Cit G Cit G Cit G Cit G K G
 or 
G G D N H G Cit G Cit G Cit G Cit G Cit G G G Cit P G A P G
G G D N H G Cit G Cit G Cit G Cit G Cit G G G Cit P G A P G
K
K
betaA
G G D N H G Cit G Cit G Cit G Cit G Cit G G G Cit P G A P G
K
G G D N H G Cit G Cit G Cit G Cit G Cit G G G Cit P G A P G
 or

GGDNHGCitGCitGCitGCitGCitGGGCitPGAPG
K
GGDNHGCitGCitGCitGCitGCitGGGCitPGAPG
K—betaA—C
GGDNHGCitGCitGCitGCitGCitGGGCitPGAPG
K
GGDNHGCitGCitGCitGCitGCitGGGCitPGAPG

In a preferred embodiment the biological sample is reacted with a combination of: or 
GA K Cit H Cit K V L Cit D N I Q G I T K P A I
K
GA K Cit H Cit K V L Cit D N I Q G I T K P A I
K —betaA—C
GA K Cit H Cit K V L Cit D N I Q G I T K P A I
K
GA K Cit H Cit K V L Cit D N I Q G I T K P A I
 and/or 
KPAICitCitLACitCitGGVKCitISGLI
K
KPAICitCitLACitCitGGVKCitISGLI
K ——betaA
KPAICitCitLACitCitGGVKCitISGLI
K
KPAICitCitLACitCitGGVKCitISGLI
 or 
KPAICitCitLACitCitGGVKCitISGLI
KPAICitCitLACitCitGGVKCitISGLI
K
K—betaA—C
KPAICitCitLACitCitGGVKCitISGLI
K
KPAICitCitLACitCitGGVKCitISGLI
 and 
G Q S Cit G Q S Cit G Cit G Cit G Cit G Cit G Cit G K G
G Q S Cit G Q S Cit G Cit G Cit G Cit G Cit G Cit G K G
K
K
betaA
G Q S Cit G Q S Cit G Cit G Cit G Cit G Cit G Cit G K G
K
G Q S Cit G Q S Cit G Cit G Cit G Cit G Cit G Cit G K G
 or 
G Q S Cit G Q S Cit G Cit G Cit G Cit G Cit G Cit G K G
K
G Q S Cit G Q S Cit G Cit G Cit G Cit G Cit G Cit G K G
K —betaA—C
G Q S Cit G Q S Cit G Cit G Cit G Cit G Cit G Cit G K G
K
G Q S Cit G Q S Cit G Cit G Cit G Cit G Cit G Cit G K G
 and/or 
G G D N H G Cit G Cit G Cit G Cit G Cit G G G Cit P G A P G
G G D N H G Cit G Cit G Cit G Cit G Cit G G G Cit P G A P G
K
K
betaA
G G D N H G Cit G Cit G Cit G Cit G Cit G G G Cit P G A P G
K
G G D N H G Cit G Cit G Cit G Cit G Cit G G G Cit P G A P G
 or

G G D N H G Cit G Cit G Cit G Cit G Cit G G G Cit P G A P G
K
G G D N H G Cit G Cit G Cit G Cit G Cit G G G Cit P G A P G
K —betaA—C
G G D N H G Cit G Cit G Cit G Cit G Cit G G G Cit P G A P G
K
G G D N H G Cit G Cit G Cit G Cit G Cit G G G Cit P G A P G

It is a further object of the invention a kit for the diagnosis of rheumatoid arthritis comprising at least two peptides as defined above.
In order to perform the assay, the peptides can be adsorbed or immobilized or covalently linked or modified with a tag or with a carrier to bind it to a solid support (e.g. chip, microsphere, bead, particle, pin, cuvette, array, tube, membrane, gold, polystyrene, reactor vessels or wells, micro-titre plate) or any other shape suitable for conducting a diagnostic method according to the invention. In a first step of the method, the sample of the biological fluid to be analyzed is placed in contact and incubated with the peptides of the invention that may be linked to the solid support. ACPA that are possibly present in the sample are thus specifically bound to the peptides of the invention, producing a peptide-antibody complex (i.e. an antigen-antibody complex). The ACPA to be detected are IgG, IgA, IgM or IgE immunoglobulins. The evaluation of the presence and/or the quantity of the antigen-antibody complex can be performed by any suitable detection method known to one skilled in the art.

In certain embodiments, the above described method may be an immunological assay.

The kit for the diagnosis of RA of the invention comprises at least two peptides as indicated above, in any combination thereof.

Preferably the kit comprises:
- peptide of formula IV or one of its MAP derivative (Formula VI or VII) and peptide of formula XI or one of its MAP derivative (Formula XII or XIII) **[Combination A1].**
- or peptide of formula V or one of its MAP derivative (Formula VIII or IX) and peptide of formula XI or one of its MAP derivative (Formula XII or XIII) **[Combination A2].**
- or peptide of formula IV or one of its MAP derivative (Formula VI or VII) and peptide of formula XV or one of its MAP derivative (Formula XVI or XVII) **[Combination B1].**
- or peptide of formula V or one of its MAP derivative (Formula VIII or IX) and peptide of formula XV or one of its MAP derivative (Formula XVI or XVII) **[Combination B2].**
- or peptide of formula V or one of its MAP derivative (Formula VIII or IX) and peptide of formula IV or one of its MAP derivative (Formula VI or VII) in combination with peptide of formula XI or one of its MAP derivative (Formula XII or XIII) **[Combination D1].**
- or peptide of formula V or one of its MAP derivative (Formula VIII or IX) and peptide of formula IV or one of its MAP derivative (Formula VI or VII) in combination with peptide of formula XV or one of its MAP derivative (Formula XVI or XVII) **[Combination E1].**
- or peptide of formula IV or one of its MAP derivative (Formula VI or VII) and peptide of formula XI or one of its MAP derivative (Formula XII or XIII) in combination with peptide of formula XV or one of its MAP derivative (Formula XVI or XVII) **[Combination G1].**
- or peptide of formula V or one of its MAP derivative (Formula VIII or IX) and peptide of formula XI or one of its MAP derivative (Formula XII or XIII) in combination with peptide of formula XV or one of its MAP derivative (Formula XVI or XVII) **[Combination G2].**
- or peptide of formula V or one of its MAP derivative (Formula VIII or IX) and peptide of formula XI or one of its MAP derivative (Formula XII or XIII) in combination with peptide of formula XV or one of its MAP derivative (Formula XVI or XVII) and with peptide of formula IV or one of its MAP derivative (Formula VI or VII) **[Combination H1].**

Still preferably the kit comprises:
- formula VI (or VII) and formula XII (or XIII) **[Combination A1a].**
- formula VIII (or IX) and formula XII (or XIII) **[Combination A2a].**
- formula VI (or VII) and formula XVI (or XVII) **[Combination B1a].**
- formula VIII (or IX) and formula XVI (or XVII) **[Combination B2a].**
- formula VIII (or IX) and formula VI (or VII) and formula XII (or XIII) **[Combination D1a].**
- formula VIII (or IX) and formula VI (or VII) and formula XVI (or XVII) **[Combination E1a].**
- formula VI (or VII) and formula XII (or XIII) in combination with formula XVI (or XVII) **[Combination G1a].**
- formula VIII (or IX) and formula XII (or XIII) in combination with MAP formula XVI (or XVII) **[Combination G2a].**
- formula VIII (or IX) and formula XII (or XIII) in combination with formula XVI (or XVII) and formula VI (or VII) **[Combination H1a].**

In particular the kit allows the detection of ACPA in biological fluids. The kit comprises at least two of the citrullinated peptides as defined above or a functional fragment thereof, and at least one further reagent. Preferably, the further reagent is an agent capable of detecting the peptide-antibody complex formed between the peptides included in the kit and the autoantibodies present in a biological sample. In the practice of the invention the detection of the peptide-antibody complex can be performed by any suitable method known to one skilled in the art.

In another embodiment of the present invention the kit as defined above comprises:
- at least two of the citrullinated peptides as defined above,
- at least one further detecting reagent,
-and a reagent for the detection of the rheumatoid factor (e.g. Fc portion of human or rabbit IgG).

The peptides included in a kit may or not be immobilized on a substrate surface (e.g. bead, array, micro-titre plate, membrane, and the like). Thus, in certain embodiments, an inventive kit may include a substrate surface.

Depending on the procedure, the kit may further comprise one or more of: dilution buffer and/or reagents, immunodetection buffer and/or reagents, and detection means. Protocols for using these buffers and reagents for performing different steps of the procedure may be included in the kit.

Preferably the method of the invention and/or the kit of the invention are for the diagnosis of rheumatoid arthritis.

Still preferably the method of the invention and/or the kit of the invention are for the detection of IgA, IgG, IgM and IgE forms of ACPA.

In the present invention the peptides are antigenically effective which means that the peptides are able to specifically bind rheumatoid arthritis-specific antibodies.

### Detailed description of the invention

The invention will be now illustrated by means of the following non limiting examples.

### Example 1. Peptide Synthesis

Peptides of the present invention may be prepared by any suitable method, including chemical synthesis and recombinant methods.
Peptides were synthesized using a Wang resin preloaded with the C-terminal amino acid of the sequence or with the MAP core and following the Fmoc/tBu solid-phase peptide strategy [R.B. Merrifield J. Am. Chem. Soc. 1963, 85, 2149; E. Atherton *et al.* Oxford: IRL Press 1989; J.P. Tam Proc. Natl. Acad. Sci. USA 1988, 85, 5409]. Fmoc deprotections were carried out in 20 min with 20% piperidine in DMF. Coupling reactions were performed by treating the resin for 45 min with a 0.5 M solution of the Fmoc-protected amino acids and HOBt in DMF (2.5 equiv), a 0.5 M solution of TBTU in DMF (2.5 equiv), and 4 M NMM in DMF (5 equiv). Peptide cleavage from the resin and deprotection of the amino acid side chains were carried out in 3 h with TFA/thioanisole/ethanedithiol/phenol/H₂O (82.5:5:2.5:5:5). The crude products were precipitated with cold Et₂O, centrifuged, and lyophilized. The pure peptides were obtained by HPLC in a purity >95% and characterized by mass spectrometry (ESI-Orbitrap and/or MALDI-TOF).

### Example 2. ELISA for the determination of anti-citrullinated peptide antibodies

At least two MAPs of the citrullinated peptide antigens according to the invention were diluted to a concentration of 1-10 µg/ml in phosphate buffered saline (PBS) and loaded into the wells of a polystyrene micro-titration plate (50 µl/well). The plate was left overnight at +4°C to permit interaction between peptide and plastics; however, it may be incubated at 37°C for 1-2 hours with the same result. Upon completion of the coating period, the wells containing the antigen, plus an equal number of wells which were used as controls, were treated for 1 hour at room temperature (RT) with 3% bovine serum albumin (BSA) in PBS. The serum samples (diluted 1:200 in a buffer constituted by 1% BSA, 0.05% Tween X-100 in PBS) were then loaded onto the plate (50 µl/well) and left to incubate for 3 hours at RT. After the incubation period, one washing was performed with 1% PBS Tween X-100 and two washings were performed with PBS (150 µl/well). An anti human-IgG, IgA, IgM or IgE antibody conjugated to the enzyme alkaline phosphatase in 1 % PBS BSA, 0.05% Tween X-100, was used to show that the antigen/antibody reaction had taken place. The antibody (50 µl/well) was then incubated for 1-3 hours at RT with agitation. Upon completion of the incubation, after three washings as described above, the alkaline phosphatase substrate (p-nitrophenyl phosphate) was added to the wells and, in the presence of the enzyme, it produced a yellow product measurable by spectrophotometric techniques at a wavelength of 405 nm; its quantity was proportional to the titre of antibodies bound. The results of the test were expressed as the percentage of positivity, calculated by dividing the absorbance of each serum sample by the absorbance of a positive serum sample the value of which was set arbitrarily at 100.
Serum samples of 108 patients suffering from RA and of 44 normal healthy subjects (NHS) were tested by this method using the peptides of combinations A1a, A2a, B1a, B2a, D1a, E1a, G1a, G2a, and H1a.
The number of RA positive sera and thus the sensitivity of each combination was determined fixing the cutoff level in order to have a specificity of 96%, data are reported in Table 1.

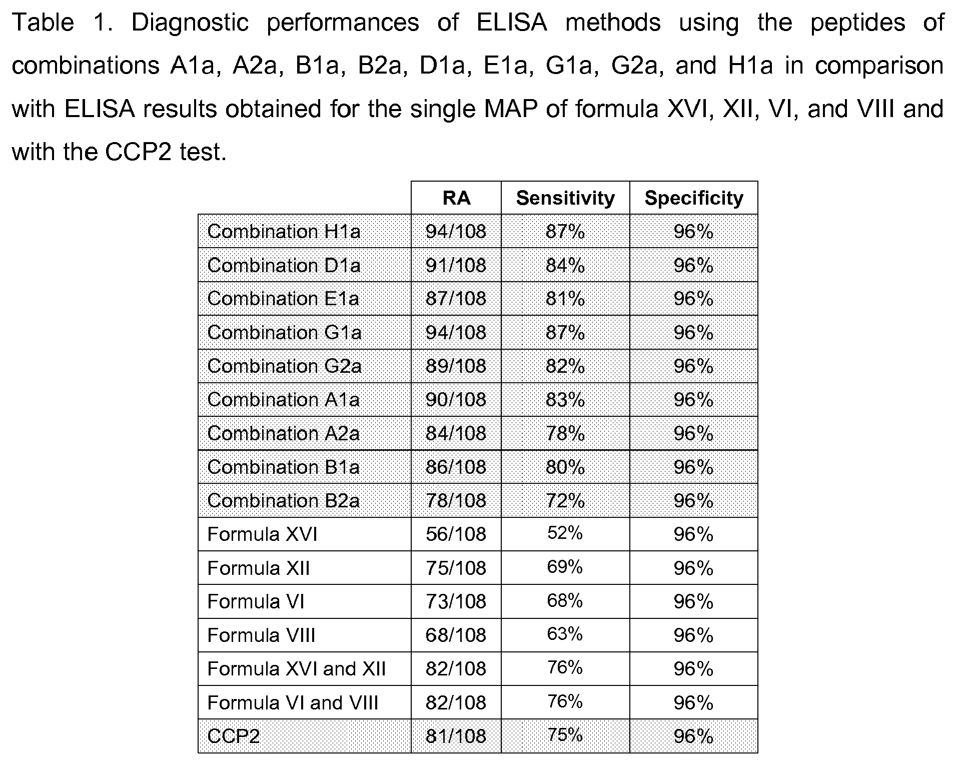

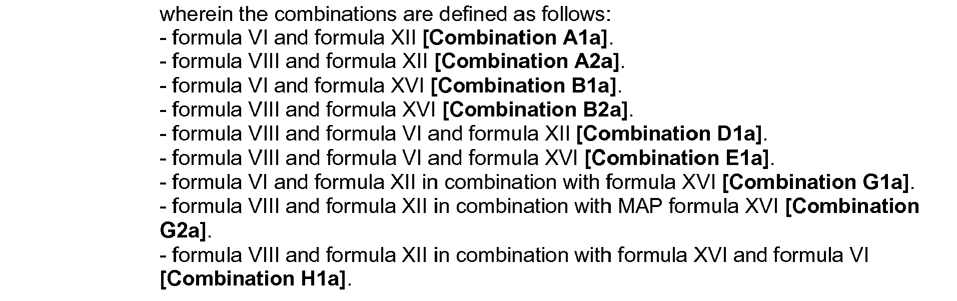

It was found that the sensitivity of the test increases using the combination of at least one sequence derived from H4(1-50) with at least one derived from one of the two EBNA proteins. In fact, the sequences derived from H4(1-50) are recognized by a sub-population of ACPA slightly different from the one identified by EBNA1 or EBNA2. Therefore, for instance, combination H1a allows the identification of 87% of RA sera that includes an 11 % of RA patients not diagnosed with the combination of only H4 peptides (Formula VI and VIII) or of only EBNA peptides (Formula XVI and XII). Moreover, almost all the combinations of the present invention are characterized by an improved sensitivity in comparison with the CCP2 test performed on the same set of sera.

Similar results were found when the MAP peptide in the form (peptide)₄ K₂ K betaA is replaced by the MAP peptide in the form (peptide)₄ K₂ K betaA-C wherein betaA is a beta-alanine residue and C is a cysteine residue.

### Example 3. Bead-based multiplex assay. xMAP® assay (Luminex Corporation) for the determination of anti-citrullinated peptide antibodies

The carboxylated beads (100 µl) were resuspended in the activation buffer (80 µl) and a solution of N-(3-dimethylaminopropyl)-N'-ethyl-carbodiimide hydrochloride (10 µl, 50 mg/ml) was added closely followed by a solution of N-hydroxysulfosuccinimide sodium salt (10 µl, 50 mg/ml). After 20 minutes beads were washed with PBS and a solution of the peptides according to the invention (5-50 µg) was added. After 2 hours at RT, or alternatively overnight at 4 °C, the beads were washed with PBS and then re-suspended with the blocking buffer (250 µl) for 30 minutes at RT. The beads were washed with the blocking buffer and then incubated with the serum samples diluted (1:50-1:200) into the blocking buffer for 1 hour at RT. After washings with the blocking buffer, the beads were treated with phycoerythrin-labeled anti human-IgG, IgA, IgM or IgE detection antibody for 1 hour at RT and then washed and resuspended in the blocking buffer. The mean fluorescence intensity was recorded with the Luminex analyzer; its quantity was proportional to the titre of antibodies bound.
Serum samples of 33 patients suffering from RA and of 44 NHS were tested by this method using the peptides of combinations A1a, A2a, B1a, B2a, D1a, E1a, G1a, G2a, and H1a in comparison with results obtained for the single MAP of formula XVI, XII, VI, and VIII.
The number of RA positive sera and thus the sensitivity of each combination was determined fixing the cutoff level in order to have a specificity of 96%, data are reported in Table 2.

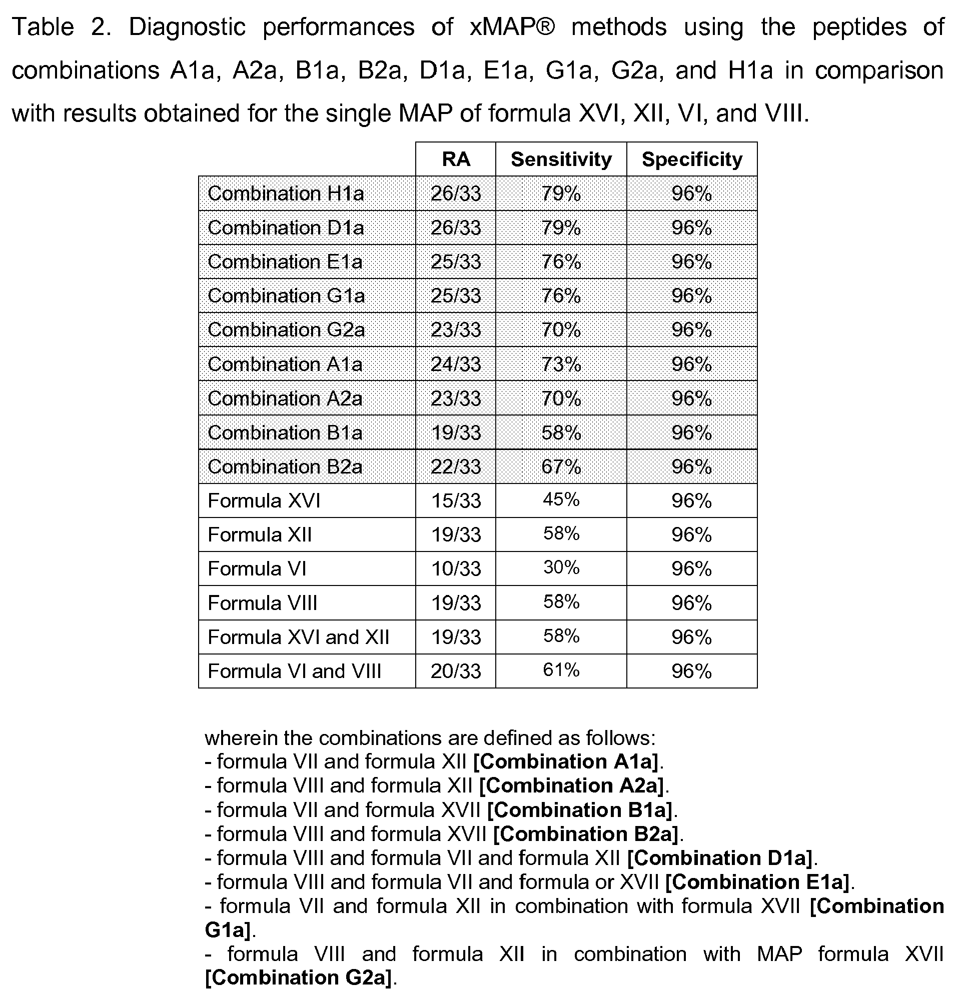

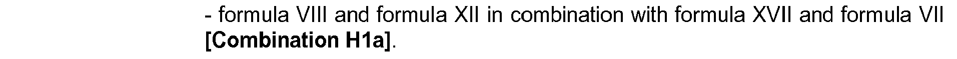

Results reported in Table 2 demonstrate that also in bead-based multiplex assays the combination of at least one sequence derived from H4(1-50) with at least one derived from one of the two EBNA proteins leads to high sensitivity diagnostic tests for RA. Therefore, data reported in Table 2 together with the ones of Table 1 demonstrate the diagnostic value of the combinations of the present invention, independently from the technology used for the detection.

Similar results were found when the MAP peptide in the form (peptide)₄ K₂ K betaA is replaced by the MAP peptide in the form (peptide)₄ K₂ K betaA-C wherein betaA is a beta-alanine residue and C is a cysteine residue.

## Claims

1. An in vitro method for the diagnosis of rheumatoid arthritis in a subject comprising the step of detecting antibodies specific for rheumatoid arthritis in a biological sample by:
a) reacting under proper conditions said biological sample with at least two peptides to produce a peptide-antibody complex, wherein
- at least one peptide consists of a sequence being at least 15 amino acid long and comprised in the sequence: 
S G X1 G K G G K G L G K G G A K X2 H X3 K V L X4 D N I Q G I T K P A I X5 X6 L
A X7 X8 G G V K X9 I S G L I (SEQ ID No. 1, Formula I)
wherein the amino acids X₁-X₉ are selected independently from an arginine residue or a citrulline residue and at least two of X₁-X₉ are a citrulline residue
and
-at least one peptide is selected from the group of:
i) a peptide consisting of a sequence being at least 8 amino acid long and comprised in the sequence: G Q S X₁ G Q S X₂ G X₃ G X₄ G X₅ G X₆ G X₇ G K G (SEQ ID No. 2, Formula X)
wherein the amino acids X₁-X₇ are selected independently from an arginine residue or a citrulline residue, and at least one of X₁-X₇ is a citrulline residue
and/or
ii) a peptide consisting of a sequence being at least 12 amino acid long and comprised in the sequence:
G G D N H G X₁ G X₂ G X₃ G X₄ G X₅ G G G X₆ P G A P G (SEQ ID No. 3, Formula XIV)
wherein the amino acids X₁-X₆ are selected independently from an arginine residue or a citrulline residue and at least one of X₁-X₆ is a citrulline residue;
b) detecting the peptide-antibody complex.

2. The method of claim 1 wherein the biological sample is reacted with at least two peptides, wherein at least one peptide consists of the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue.

3. The method of claim 1 or 2 wherein the biological sample is reacted with at least two peptides, wherein at least one peptide consists of the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue.

4. The method of any one of previous claim wherein the biological sample is reacted with at least two peptides, wherein at least one peptide consists of the sequence G G D N H G X₁ G X₂ G X₃ G X₄ G X₅ G G G X₆ P G A P G (SEQ ID No.3, Formula XIV) wherein the amino acids X₁-X₆ are selected independently from an arginine residue or a citrulline residue and at least one of X₁-X₆ is a citrulline residue.

5. The method of any one of previous claim wherein the biological sample is reacted with:
a) two peptides, wherein
-one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-one peptide is selected from the group of: i) a peptide consisting of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
or
ii) a peptide consisting of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue or
b) three peptides, wherein
-one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-two peptides consist of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue or
c) three peptides, wherein
-two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-one peptide is selected from the group of:
i) a peptide consisting of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
or
ii) a peptide consisting of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue
or
d) three peptides, wherein
-one peptide consists of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-one peptide consists of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
and
-one peptide consists of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue
or
e) four peptides, wherein
-two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-two peptides consist of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue
or
f) four peptides, wherein
-two peptides consist of a sequence being at least 15 amino acid long and comprised in the sequence S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I) wherein the amino acids X₁-X₉ are selected independently from an arginine residue or a citrulline residue and at least two of X₁-X₉ are a citrulline residue
and
-one peptide consists of a sequence being at least 8 amino acid long and comprised in the sequence G Q S X₁ G Q S X₂ G X₃ G X₄ G X₅ G X₆ G X₇ G K G (SEQ ID No. 2, Formula X) wherein the amino acids X₁-X₇ are selected independently from an arginine residue or a citrulline residue, and at least one of X₁-X₇ is a citrulline residue and
-one peptide consists of a sequence being at least 12 amino acid long and comprised in the sequence G G D N H G X₁ G X₂ G X₃ G X₄ G X₅ G G G X₆ P G A P G (SEQ ID No. 3, Formula XIV) wherein the amino acids X₁-X₆ are selected independently from an arginine residue or a citrulline residue and at least one of X₁-X₆ is a citrulline residue.

6. The method according to any one of previous claim wherein
- the at least one peptide consisting of a sequence being at least 15 amino acid long and comprised in the sequence of SEQ ID No. 1 is selected from the group of:
- S G X₁ G K G G K G L G K G G A (aa 1 to aa 15 of SEQ ID No. 1),
- S G X₁ G K G G K G L G K G G A K X₂ H X₃ (aa 1 to aa 20 of SEQ ID No. 1)
- G A K X₂ H X₃ K V L X₄ D N I Q G (aa 14 to aa 28 of SEQ ID No.1),
- G A K X₂ H X₃ K V L X₄ D N I Q G I T K P A I (aa 14 to aa 34 of SEQ ID No. 1)
- X₃ K V L X₄ D N I Q G I T K P A I X₅ X₆ L A (aa 19 to aa 38 of SEQ ID No. 1),
- L X₄ D N I Q G I T K P A I X₅ X₆ L A X₇ X₈ G (aa 22 to aa 41 of SEQ ID No. 1),
- K P A I X₅ X₆ L A X₇ X₈ G G V K X₉ I S G L I (aa 31 to aa 50 of SEQ ID No. 1)
or
- I X₅ X₆ L A X₇ X₈ G G V K X₉ I S G (aa 34 to aa 48 of SEQ ID No. 1)
wherein the amino acids X₁-X₉ are selected independently from an arginine residue or a citrulline residue and at least two of X₁-X₉ are a citrulline residue.

7. The method according to claim 6 wherein the at least one peptide is selected from the group of:
- G A K Cit H R K V L Cit D N I Q G I T K P A I (aa 14 to aa 34 of SEQ 10 No.1 wherein X₂ = X₄ = Cit and X₃ = Arg)
- K P A I Cit X₆ LA X7 Cit G G V K X₉ I S G L I (aa 31 to aa 50 of SEQ ID No. 1 wherein X₅ = X₈ = Cit and X₆, X₇ and X₉ = are selected independently from an arginine residue (Arg) or a citrulline (Cit) residue)
- K P A I Cit R L A R Cit G G V K R I S G L I (aa 31 to aa 50 of SEQ ID No. 1 wherein X₅ = X₈ = Cit and X₆ = X₇ = X₉ = Arg)
- K P A I R Cit L A R Cit G G V K R I S G L I (aa 31 to aa 50 of SEQ ID No. 1 wherein X₆ = X₈ = Cit and X₅ = X₇ = X₉ = Arg) 
-S G Cit G K G G K G L G K G G A K Cit H Cit K V L Cit D N I Q G I T K P A I Cit
Cit L A Cit Cit G G V K Cit I S G L I (SEQ ID No. 1 wherein X1 = X2= X3= X4 = X5
=X6 = X7=X8 =X9= Cit, Formula II)
- S G Cit G K G G K G L G K G G A K Cit H Cit K (aa 1 to aa 20 of SEQ ID No. 1 wherein X₁ = X₂ = X₃ = Cit, Formula III),
- G A K Cit H Cit K V L Cit D N I Q G I T K P A I (aa 14 to aa 34 of SEQ ID No. 1 wherein X₂ = X₃ = X₄ = Cit, Formula IV),
- K P A I Cit Cit L A Cit Cit G G V K Cit I S G L I (aa 31 to aa 50 of SEQ ID No. 1 wherein X₅ = X₆ = X₇ = X₈ = X₉ = Cit, Formula V)
- S G Cit G K G G K G L G K G G A (aa 1 to aa 15 of SEQ ID No. 1 wherein X₁ = Cit),
- G A K Cit H Cit K V L Cit D N I Q G (aa 14 to aa 28 of SEQ ID No. 1 wherein X₂ = X₃ = X₄ = Cit),
- Cit K V L Cit D N I Q G I T K P A I Cit Cit L A (aa 19 to aa 38 of SEQ ID No. 1 wherein X₃ = X₄ = X₅ = X₆ = Cit),
- L Cit D N I Q G I T K P A I Cit Cit L A Cit Cit G (aa 22 to aa 41 of SEQ ID No. 1 wherein X₄ = X₅ = X₆ = X₇ = X₈ = Cit) or
- I Cit Cit L A Cit Cit G G V K Cit I S G (aa 34 to aa 48 of SEQ ID No. 1 wherein X₅ = X₆ = X₇ = X₈ = X₉ = Cit).

8. The method according to any one of previous claim wherein the at least one peptide consisting of a sequence being at least 8 amino acid long and comprised in the sequence of SEQ ID No. 2 is selected from the group of:
- G Q S R G Q S Cit G R G R G R G R G R G K G (SEQ ID No. 2 wherein X₂ = Cit and X₁ = X₃ = X₄ = X₅ = X₆ = X₇ = Arg)
- G Q S R G Q S R G R G Cit G R G R G R G K G (SEQ ID No. 2 wherein X₄ = Cit and X₁ = X₂ = X₃ = X₅ = X₆ = X₇ = Arg)
- G Q S R G Q S Cit G R G Cit G R G R G R G K G (SEQ ID No. 2 wherein X₂ = X₄ = Cit and X₁ = X₃ = X₅ = X₆ = X₇ = Arg)
- G Q S Cit G Q S Cit G R G Cit G R G R G R G K G (SEQ ID No. 2 wherein X₁ = X₂ = X₄ = Cit and X₃ = X₅ = X₆ = X₇ = Arg)
- G Q S Cit G Q S Cit G Cit G Cit G R G R G R G K G (SEQ ID No. 2 wherein X₁ = X₂ = X₃ = X₄ = Cit and X₅ = X₆ = X₇ = Arg)
- G Q S Cit G Q S Cit G R G Cit G Cit G R G R G K G (SEQ ID No. 2 wherein X₁ = X₂ = X₄ = X₅ = Cit and X₃ = X₆ = X₇ = Arg)
- G Q S Cit G Q S Cit G Cit G Cit G Cit G R G R G K G (SEQ ID No. 2 wherein X₁ = X₂ = X₃ = X₄ = X₅ = Cit and X₆ = X₇ = Arg)
- G Q S Cit G Q S Cit G Cit G Cit G R G Cit G R G K G (SEQ ID No. 2 wherein X₁ = X₂ = X₃ = X₄ = X₆ = Cit and X₅ = X₇ = Arg)
- G Q S Cit G Q S Cit G Cit G Cit G Cit G Cit G R G K G (SEQ ID No. 2 wherein X₁ = X₂ = X₃ = X₄ = X₅ = X₆ = Cit and X₇ = Arg)
- G Q S Cit G Q S Cit G Cit G Cit G R G Cit G Cit G K G (SEQ ID No. 2 wherein X₁ = X₂ = X₃ = X₄ = X₆ = X₇ = Cit and X₅ = Arg) or
- G Q S Cit G Q S Cit G Cit G Cit G Cit G Cit G Cit G K G (SEQ ID No. 2 wherein all of X₁-X₇ are citrulline residues Formula XI)
- G Q S Cit G Q S Cit (aa 1 to aa 8 of SEQ ID No. 2 wherein all of X₁-X₂ are citrulline residues);
- G Q S Cit G Q S Cit G (aa 1 to aa 9 of SEQ ID No. 2 wherein all of X₁-X₂ are citrulline residues);
- G Cit G Cit G Cit G Cit G Cit G K G (aa 9 to aa 21 of SEQ ID No. 2 wherein all of X₃-X₇ are citrulline residues).

9. The method according to any one of previous claim wherein the at least one peptide consisting of a sequence being at least 12 amino acid long and comprised in the sequence of SEQ ID No. 3 is selected from the group of:
- G X₁ G X₂ G X₃ G X₄ G X₅ G G (aa 6 to aa 17 of SEQ ID No. 3) wherein the amino acids X₁-X₉ are selected independently from an arginine residue or a citrulline residue and at least one of X₁-X₅ is a citrulline residue;
- G X₁ G X₂ G X₃ G X₄ G X₅ G G G X₆ P G (aa 6 to aa 21 of SEQ ID No. 3) wherein the amino acids X₁-X₆ are selected independently from an arginine residue or a citrulline residue and at least one of X₁-X₅ is a citrulline residue;
- G Cit G Cit G Cit G Cit G Cit G G (aa 6 to aa 17 of SEQ ID No. 3 wherein all of X₁-X₅ are citrulline residues);
- G Cit G Cit G Cit G Cit G Cit G G G Cit P G (aa 6 to aa 21 of SEQ ID No. 3 wherein all of X₁-X₆ are citrulline residues);
- G G D N H G Cit G Cit G Cit G Cit G Cit G G G Cit P G A P G (SEQ ID No. 3 wherein all of X₁-X₆ are citrulline residues, Formula XV).

10. The method according to any one of previous claim wherein the biological sample is reacted with a combination of:
- G A K Cit H Cit K V L Cit D N I Q G I T K P A I (aa 14 to aa 34 of SEQ ID No. 1 wherein X₂ = X₃ = X₄ = Cit
and/or
- K P A I Cit Cit L A Cit Cit G G V K Cit I S G L I (aa 31 to aa 50 of SEQ ID No. 1 wherein X₅ = X₆ = X₇ = X₈ = X₉ = Cit
and
- G Q S Cit G Q S Cit G Cit G Cit G Cit G Cit G Cit G K G (SEQ ID No. 2 wherein all of X₁-X₉ are citrulline residues
and/or
- G G D N H G Cit G Cit G Cit G Cit G Cit G G G Cit P G A P G (SEQ ID No. 3 wherein all of X₁-X₆ are citrulline residues

11. The method of any one of previous claim wherein the peptides are in a map form: (peptide)₄ K₂ K betaA or (peptide)₄ K₂ K betaA-C wherein betaA is a beta-alanine residue and C is a cysteine residue.

12. The method of claim 11 wherein at least one peptide is selected from the group of: 
GAKCitHCitKVLCitDNIQGITKPAI
K
GAKCitHCitKVLCitDNIQGITKPAI
K——betaA
GAKCitHCitKVLCitDNIQGITKPAI
K
GAKCitHCitKVLCitDNIQGITKPAI
 or 
G A K Cit H Cit K V L Cit D N I Q G I T K P A I
K
G A K Cit H Cit K V L Cit D N I Q G I T K P A I
K —betaA—C
G A K Cit H Cit K V L Cit D N I Q G I T K P A I
K
G A K Cit H Cit K V L Cit D N I Q G I T K P A I
 or 
KPAICitCitLACitCitGGVKCitISGLI
K
KPAICitCitLACitCitGGVKCitISGLI
K —— betaA
KPAICitCitLACitCitGGVKCitISGLI
K
KPAICitCitLACitCitGGVKCitISGLI
 or 
KPAICitCitLACitCitGGVKCitISGLI
K
KPAICitCitLACitCitGGVKCitISGLI
K—betaA—C
KPAICitCitLACitCitGGVKCitISGLI
K
KPAICitCitLACitCitGGVKCitISGLI
 or 
G Q S Cit G Q S Cit G Cit G Cit G Cit G Cit G Cit G K G
G Q S Cit G Q S Cit G Cit G Cit G Cit G Cit G Cit G K G
G Q S Cit G Q S Cit G Cit G Cit G Cit G Cit G Cit G K G
G Q S Cit G Q S Cit G Cit G Cit G Cit G Cit G Cit G K G
K
K
K
betaA
 or 
G Q S Cit G Q S Cit G Cit G Cit G Cit G Cit G Cit G K G
K
G Q S Cit G Q S Cit G Cit G Cit G Cit G Cit G Cit G K G
K —betaA—C
G Q S Cit G Q S Cit G Cit G Cit G Cit G Cit G Cit G K G
K
G Q S Cit G Q S Cit G Cit G Cit G Cit G Cit G Cit G K G
 or 
G G D N H G Cit G Cit G Cit G Cit G Cit G G G Cit P G A P G
K
G G D N H G Cit G Cit G Cit G Cit G Cit G G G Cit P G A P G
K —— betaA
G G D N H G Cit G Cit G Cit G Cit G Cit G G G Cit P G A P G
K
G G D N H G Cit G Cit G Cit G Cit G Cit G G G Cit P G A P G
 or

G G D N H G Cit G Cit G Cit G Cit G Cit G G G Cit P G A P G
K
G G D N H G Cit G Cit G Cit G Cit G Cit G G G Cit P G A P G
K —betaA—C
G G D N H G Cit G Cit G Cit G Cit G Cit G G G Cit P G A P G
K
G G D N H G Cit G Cit G Cit G Cit G Cit G G G Cit P G A P G

13. The method according to any one of previous claim wherein the biological sample is reacted with a combination of:
group of: 
GAKCitHCitKVLCitDNIQGITKPAI
K
GAKCitHCitKVLCitDNIQGITKPAI
K —betaA
GAKCitHCitKVLCitDNIQGITKPAI
K
GAKCitHCitKVLCitDNIQGITKPAI
 or 
GA K Cit H Cit K V L Cit D N I Q G I T K P A I
K
GA K Cit H Cit K V L Cit D N I Q G I T K P A I
K —betaA— C
GA K Cit H Cit K V L Cit D N I Q G I T K P A I
K
GA K Cit H Cit K V L Cit D N I Q G I T K P A I
 and/or 
KPAICitCitLACitCitGGVKCitISGLI
KPAICitCitLACitCitGGVKCitISGLI
KPAICitCitLACitCitGGVKCitISGLI
KPAICitCitLACitCitGGVKCitISGLI
K
K
K
betaA
 or and 
G Q S Cit G Q S Cit G Cit G Cit G Cit G Cit G Cit G K G
G Q S Cit G Q S Cit G Cit G Cit G Cit G Cit G Cit G K G
K
K
betaA
G Q S Cit G Q S Cit G Cit G Cit G Cit G Cit G Cit G K G
K
G Q S Cit G Q S Cit G Cit G Cit G Cit G Cit G Cit G K G
 or 
G Q S Cit G Q S Cit G Cit G Cit G Cit G Cit G Cit G K G
K
G Q S Cit G Q S Cit G Cit G Cit G Cit G Cit G Cit G K G
K —betaA—C
G Q S Cit G Q S Cit G Cit G Cit G Cit G Cit G Cit G K G
K
G Q S Cit G Q S Cit G Cit G Cit G Cit G Cit G Cit G K G
 and/or 
G G D N H G Cit G Cit G Cit G Cit G Cit G G G Cit P G A P G
G G D N H G Cit G Cit G Cit G Cit G Cit G G G Cit P G A P G
G G D N H G Cit G Cit G Cit G Cit G Cit G G G Cit P G A P G
G G D N H G Cit G Cit G Cit G Cit G Cit G G G Cit P G A P G
K
K
K
betaA
 or

G G D N H G Cit G Cit G Cit G Cit G Cit G G G Cit P G A P G
K
G G D N H G Cit G Cit G Cit G Cit G Cit G G G Cit P G A P G
K —betaA—C
G G D N H G Cit G Cit G Cit G Cit G Cit G G G Cit P G A P G
K
G G D N H G Cit G Cit G Cit G Cit G Cit G G G Cit P G A P G

14. A kit for the diagnosis of rheumatoid arthritis comprising at least two peptides as defined in any one of previous claims.


SEQUENCE LISTING

<110> Toscana Biomarkers srl
PRATESI, Federico
ALCARO, Maria Claudia
CHELLI, Mario
LOLLI, Francesco
PAOLINI, Ilaria
PAPINI, Anna Maria
ROVERO, Paolo
MIGLIORINI, Paola

<120> METHOD FOR THE DIAGNOSIS OF RHEUMATOID ARTHRITIS

<130> BE 112405

<160> 4

<170> PatentIn version 3.5

<210> 1
<211> 50
<212> PRT
<213> Artificial Sequence

<220>
<223> synthetic peptide


<220>
<221> MISC_FEATURE
<222> (3)..(3)
<223> arginine or citrulline residue

<220>
<221> MISC_FEATURE
<222> (17)..(17)
<223> arginine or citrulline residue

<220>
<221> MISC_FEATURE
<222> (19)..(19)
<223> arginine or citrulline residue

<220>
<221> MISC_FEATURE
<222> (23)..(23)
<223> arginine or citrulline residue

<220>
<221> MISC_FEATURE
<222> (35)..(35)
<223> arginine or citrulline residue

<220>
<221> MISC_FEATURE
<222> (36)..(36)
<223> arginine or citrulline residue

<220>
<221> MISC_FEATURE
<222> (39)..(39)
<223> arginine or citrulline residue

<220>
<221> MISC_FEATURE
<222> (40)..(40)
<223> arginine or citrulline residue

<220>
<221>  MISC_FEATURE
<222>  (45)..(45)
<223>  arginine or citrulline residue

<400>  1

Ser Gly Xaa Gly Lys Gly Gly Lys Gly Leu Gly Lys Gly Gly Ala Lys
1               5                   10                  15

Xaa His Xaa Lys Val Leu Xaa Asp Asn Ile Gln Gly Ile Thr Lys Pro
            20                  25                  30

Ala Ile Xaa Xaa Leu Ala Xaa Xaa Gly Gly Val Lys Xaa Ile Ser Gly
        35                  40                  45

Leu Ile
    50

<210>  2
<211>  21
<212>  PRT
<213>  Artificial Sequence

<220>
<223>  synthetic peptide

<220>
<221>  MISC_FEATURE
<222>  (4)..(4)
<223>  arginine or citrulline residue

<220>
<221>  MISC_FEATURE
<222>  (8)..(8)
<223>  arginine or citrulline residue

<220>
<221>  MISC_FEATURE
<222>  (10)..(10)
<223>  arginine or citrulline residue

<220>
<221>  MISC_FEATURE
<222>  (12)..(12)
<223>  arginine or citrulline residue

<220>
<221>  MISC_FEATURE
<222>  (14)..(14)
<223>  arginine or citrulline residue

<220>
<221>  MISC_FEATURE
<222>  (16)..(16)
<223>  arginine or citrulline residue

<220>
<221>  MISC_FEATURE
<222>  (18)..(18)
<223>  arginine or citrulline residue

<400>  2

Gly Gln Ser Xaa Gly Gln Ser Xaa Gly Xaa Gly Xaa Gly Xaa Gly Xaa
1               5                   10                  15

Gly Xaa Gly Lys Gly
            20

<210>  3
<211>  24
<212>  PRT
<213>  Artificial Sequence

<220>
<223>  synthetic peptide

<220>
<221>  MISC_FEATURE
<222>  (7)..(7)
<223>  arginine or citrulline residue

<220>
<221>  MISC_FEATURE
<222>  (9)..(9)
<223>  arginine or citrulline residue

<220>
<221>  MISC_FEATURE
<222>  (11)..(11)
<223>  arginine or citrulline residue

<220>
<221>  MISC_FEATURE
<222>  (13)..(13)
<223>  arginine or citrulline residue

<220>
<221>  MISC_FEATURE
<222>  (15)..(15)
<223>  arginine or citrulline residue

<220>
<221>  MISC_FEATURE
<222>  (19)..(19)
<223>  arginine or citrulline residue

<400>  3

Gly Gly Asp Asn His Gly Xaa Gly Xaa Gly Xaa Gly Xaa Gly Xaa Gly
1               5                   10                  15

Gly Gly Xaa Pro Gly Ala Pro Gly
            20

<210>  4
<211>  21
<212>  PRT
<213>  Artificial Sequence

<220>
<223>  synthetic peptide

<400>  4

Gly Gln Ser Arg Gly Gln Ser Arg Gly Arg Gly Arg Gly Arg Gly Arg
1               5                   10                  15

Gly Arg Gly Lys Gly
            20